# EUROPEAN PATENT SPECIFICATION

(11) **EP 3 370 618 B1**
(45) Date of publication and mention of the grant of the patent: **25.12.2019**
(21) Application number: 15801230.2
(22) Date of filing: 03.11.2015
(51) Int. Cl.: A61B 6/02, A61B 6/06, A61B 6/14, A61B 6/00

(54) **DYNAMIC IMAGE DATA TREATMENT IN DENTAL IMAGING DEVICES**
DYNAMISCHE BILDDATENBEHANDLUNG BEI ZAHNÄRZTLICHEN BILDGEBUNGSVORRICHTUNGEN
TRAITEMENT DYNAMIQUE DES DONNÉES D'IMAGES DANS DES DISPOSITIFS D'IMAGERIE DENTAIRE

(43) Date of publication of application: 12.09.2018
(73) Proprietor: Trophy, 77435 Marne La Vallée Cedex 2 (FR)
(72) Inventor: COMPARETTI, Chloe Abdoul Carime, 77435 Marne la Vallee Cedex 2 (FR); VLACHOMITROU, Anna-Sesilla, 77435 Marne la Vallee Cedex 2 (FR); LOUSTAUNEAU, Vincent, 77435 Marne la Vallee Cedex 2 (FR)
(74) Representative: Wagner & Geyer
(86) International application number: PCT/IB2015/002201
(87) International publication number: WO 2017/077354

(56) References cited:
- US-A1- 2009 196 395
- US-A1- 2012 243 662
- US-A1- 2014 226 793

## Description

### FIELD OF THE INVENTION

The invention relates generally to the field of medical x-ray imaging and more particularly to the field of cephalometric dental x-ray imaging. Further, the invention relates to a combined cephalometric, panoramic and computed tomography dental imaging apparatus and/or methods.

### BACKGROUND

In the field of dental imaging, cephalometric images (or skull images) are helpful for example in orthodontics or for any type of skull analysis. Commonly, cephalometric imaging necessitates the use of a sensor sized to fit the size of the skull. One single x-ray shot can then image the skull. The price of such a sensor corresponds to an important part of the price of the entire cephalometric imaging device. In order to decrease the price of the imaging device, it is possible to use an elongated sensor, or in other words, a line detector camera. Then a scan of the object (skull) is performed by acquiring a plurality of thin elongated frames. The frames are then stitched together to form the cephalometric image of the skull.

In the standard art, a primary collimator that may be a blade or shutter collimator is positioned in front of the x-ray source to roughly shape a slit x-ray beam. A secondary collimator is positioned on the side of a cephalometric imaging module at the end of a cephalometric arm. The secondary collimator aims at more precisely shaping the x-ray beam that radiates a slit imaging device (e.g., digital detector) after traversing a patient's head positioned on a patient's positioner between the secondary collimator and the slit imaging device. In the standard art, it is essential to realize a perfect alignment between the focal point of the X-ray source, the center of the apertures of the primary and secondary collimators and the center of the elongated slit imaging device at any step of the scanning process. During the scan of the skull, the aperture of the primary collimator is translated at a monitored speed. The secondary collimator and the slit imaging device slide along paralleled rails and are mechanically coupled by a coupling mechanism so that their speed of displacement are correlated in a constant ratio.

Furthermore, reference is made to US 2014 0 226 793 A1 disclosing an automatic field-of-view size calculation constraint for systems and methods for generating an image. One system includes a processor configured to receive a requested object field-of-view and determine if the object field-of-view is possible based on constraints of an imaging system, wherein the constraints include dimensions of a detector panel and motion limits of a collimator assembly. If the object field-of-view is possible, the processor is configured to dynamically generate a set of collimator motor commands and reconstruction parameters based on the object field-of-view, calibration parameters of the collimator assembly and geometric calibration parameters of the imaging system. The processor is also configured to provide the set of collimator motor commands to the collimator assembly to position shutters of the collimator assembly to illuminate the object field-of-view.

While such systems may have achieved certain degrees of success in their particular applications, there is consequently a need for improving the quality of the cephalometric image.

### SUMMARY

In accordance with the present invention, a method of x-ray imaging of a region of interest of a patient with an X-ray apparatus and an X-ray apparatus to image a region of interest of an imaging area of the apparatus as set forth in Claims 1 and 15, respectively, is provided. Further embodiments of the invention are inter alia disclosed in the dependent claims. An aspect of this application is to advance the art of medical digital radiography, particularly for dental applications.

Another aspect of this application is to address, in whole or in part, at least the foregoing and other deficiencies in the related art.

It is another aspect of this application to provide, in whole or in part, at least the advantages described herein.

An advantage offered by apparatus and/or method embodiments of the application relates to improved scan imaging such as panoramic dental scan imaging or cephalometric dental scan imaging.

Another advantage of exemplary method and/or apparatus embodiments according to the application relates to providing dynamic cropping for a cropped area on an active area of the detector for each frame obtained during a scan imaging.

According to one aspect of the disclosure, there is provided a method of x-ray imaging with an x-ray apparatus that can include performing a first scan imaging of a region of interest with an aperture of at least one collimator and at least one of an x-ray source and an x-ray imaging device following a first scan trajectory; collecting a plurality of frames from the first scan imaging of the region of interest; determining a location of at least one edge of an irradiated area on at least one frame of the region of interest from said plurality of frames from said first scan imaging; establishing an edge position curve relative to the first scan imaging of the region of interest; cropping selected frames of said plurality of frames from said first scan imaging on the basis of said edge position curve; calculating an actual exposure profile relative to the first scan imaging of the region of interest; and reconstructing the region of interest by combining said cropped selected frames using said actual exposure profile.

These aspects are given only by way of illustrative example, and such objects may be exemplary of one or more embodiments of the invention. Other desirable objectives and advantages inherently achieved by the disclosed invention may occur or become apparent to those skilled in the art. The invention is defined by the appended claims.

### BRIEF DESCRIPTION OF THE DRAWINGS

The foregoing and other objects, features, and advantages of the invention will be apparent from the following more particular description of the embodiments of the invention, as illustrated in the accompanying drawings.

The elements of the drawings are not necessarily to scale even relative to each other. Some exaggeration may be necessary in order to emphasize basic structural relationships or principles of operation. Some conventional components that would be needed for implementation of the described embodiments, such as support components used for providing power, for packaging, and for mounting and protecting system optics, for example, are not shown in the drawings in order to simplify description.
Fig. 1 is a diagram that shows an imaging device for producing cephalometric images and/or panoramic images that can implement method and/or apparatus embodiments according to the application.
Fig. 2 is a diagram that shows a top view illustrating alignment of the center of the primary collimator, the center of the secondary collimator and the center of the sensor during a cephalometric scan.
Fig. 3 is a diagram that shows a top view illustrating the translation of the center of the primary collimator, the center of the secondary collimator and the center of the sensor during a cephalometric scan.
Fig. 4 is an illustration of an exemplary mechanism to synchronize motion of a secondary collimator and an imaging sensor.
Fig. 5a is a diagram that shows a situation where the radiated area is centered on the active area of the imaging sensor because of correct alignment of a secondary collimator with an imaging sensor.
Fig.5b is a diagram that shows a situation where the central axis of an X-ray radiated area is transversally offset relative to the central axis of an active area of an imaging sensor.
Fig. 5c is a diagram that shows a situation where the central axis of the X-ray radiated area is transversally offset relative to the central axis of the active area of the imaging sensor so that the radiated area extends partially out of a cropped imaging area of the imaging sensor.
Fig. 6 is a diagram that shows ordered and actual edge position curves relative to an exemplary cephalometric imaging scan.
Fig. 7a is a diagram that shows a non corrected exposure profile and an actual exposure profile of a cephalometric scan, this latter being calculated from the edge position curve of Fig. 7b and the non corrected exposure profile.
Fig. 7b is a diagram that shows ordered and actual edge position curves relative to the implementation of a first exemplary cephalometric imaging method embodiment according to the application.
Fig. 8 is a flowchart that shows a first exemplary cephalometric imaging method embodiment according to the application.
Fig. 9 is a diagram that shows a frame imaged during a scan of a patient.
Fig. 10 is a diagram that shows an exemplary dynamic crop of a frame, the cropped area being defined by an actual position of a lateral edge of the radiated area of an imaging detector.
Fig. 11.1 is a diagram that shows a cephalometric image reconstruction using the non corrected exposure profile without implementing embodiments according to the application.
Fig. 11.2 is a diagram that shows a cephalometric image reconstruction using the actual exposure profile calculated using an exemplary dynamic image cropping embodiment according to the application.
Fig. 12 is a flowchart that shows another exemplary cephalometric imaging method embodiment according to the application.
Fig. 13 is a diagram that shows an ordered edge position curve and calibration edge position curves relative to the blank scan and edge calibration curve relative to the subsequent patient's scan in an exemplary cephalometric imaging method embodiment according to the application.
Fig. 14 is a diagram that shows a non corrected exposure profile and an actual exposure profile of the patient's cephalometric scan.
Fig. 15 is a diagram that shows exemplary alternative profiles for an actual edge position curve of a trajectory for a scan imaging.
Fig. 16 is a diagram that shows a non corrected exposure profile of a dental panoramic scan obtained after feedback adjustment from the preset trajectory.
Fig. 17 is a flowchart that shows an exemplary panoramic imaging method embodiment according to the application.
Fig. 18 is a diagram that shows an ordered edge position curve (curve 1) and a calibration edge position curve relative to the blank scan (curve 2) and an edge position curve relative to the subsequent patient's scan (curve 3) in a panoramic imaging method.
Fig. 19 is a diagram that shows a non corrected exposure profile and actual exposure profiles of the patient's panoramic scan.

### DESCRIPTION OF EXEMPLARY EMBODIMENTS

The following is a description of exemplary embodiments of the application, reference being made to the drawings in which the same reference numerals identify the same elements of structure in each of the several figures.

In the following description, a certain exemplary embodiments of the application will be described as an algorithm or software program. Those skilled in the art will recognize that the equivalent of such software may also be constructed in hardware. Because image manipulation algorithms and systems are well known, the present description will be directed in particular to algorithms and systems forming part of, or cooperating more directly with, the method in accordance with the present invention. Other aspects of such algorithms and systems, and hardware and/or software for producing and otherwise processing the image signals involved therewith, not specifically shown or described herein may be selected from such systems, algorithms, components and elements known in the art.

A computer program product may include one or more storage medium, for example; magnetic storage media such as magnetic disk (such as a floppy disk) or magnetic tape; optical storage media such as optical disk, optical tape, or machine readable bar code; solid-state electronic storage devices such as random access memory (RAM), or read-only memory (ROM); or any other physical device or media employed to store a computer program having instructions for controlling one or more computers to practice the method according to the present invention.

Exemplary method embodiments described herein may be described with reference to a flowchart. Describing exemplary methods by reference to a flowchart enables one skilled in the art to develop such programs, firmware, or hardware, including such instructions to carry out the methods on suitable computers, executing the instructions from computer-readable media. Similarly, exemplary methods performed by the service computer programs, firmware, or hardware are also composed of computer-executable instructions.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim.

In the following claims, the terms "first," "second," and "third," and the like, are used merely as labels, and are not intended to impose numerical requirements on their objects.

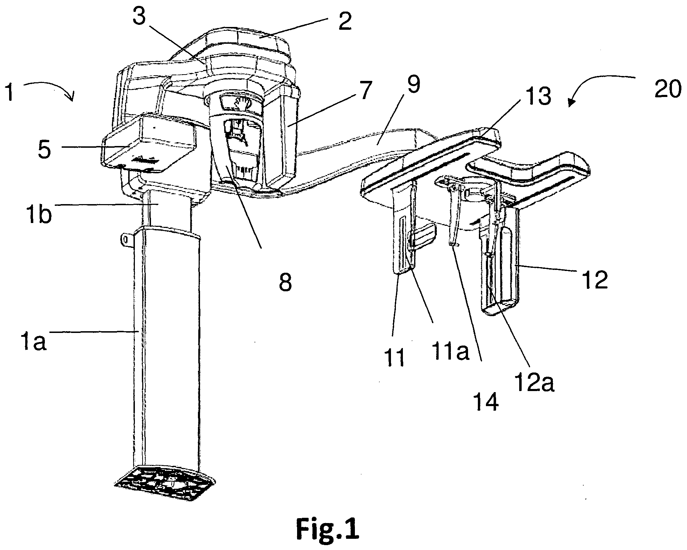

Fig. 1 is a diagram that shows an imaging device for producing cephalometric images and/or panoramic images that can implement exemplary method and/or apparatus embodiments according to the invention. As shown in Fig. 1, a cephalometric imaging device 1 can include a vertical column including a lower part 1a and an upper part 1b that is slidable relative to the lower part to adjust the patient's height. A platform 2 is positioned at the top of the upper part 1b of the vertical column and supports a gantry 3. The gantry 3 supports an X-ray source 5 with a primary collimator 10 to shape the x-ray beam originating from the source. In one embodiment, the primary collimator is a blade collimator or a shutter collimator. In an exemplary embodiment, the collimator shapes the beam into a rectangular slit beam. In an alternative embodiment, cross section of the beam may have the shape of a lozenge or a trapeze. Optionally, an imaging device 7 is supported by the gantry to acquire a panoramic and/or a CT image and a patient support 8 aims at repeatedly positioning a patient for the panoramic and/or CT imaging. In one embodiment, the X-ray source 5 and the imaging device are located at opposing extremities of the gantry. An elongated cephalometric arm 9 can extend from the upper slidable part 1b of the column and support a cephalometric imaging module 20. This cephalometric module 20 can include a cephalometric platform 13 supporting a patient support 14 to position a patient 1000 between the secondary collimator 11 and an imaging detector 12 for cephalometric imaging. In one exemplary embodiment, the secondary collimator 11 is a plate provided with an elongated slit-shaped aperture and the imaging detector 12 is a line detector, or slit-shaped detector, composed of an elongated area of pixels. The line detector used for the detector 12 can be a few tens of pixels in width.

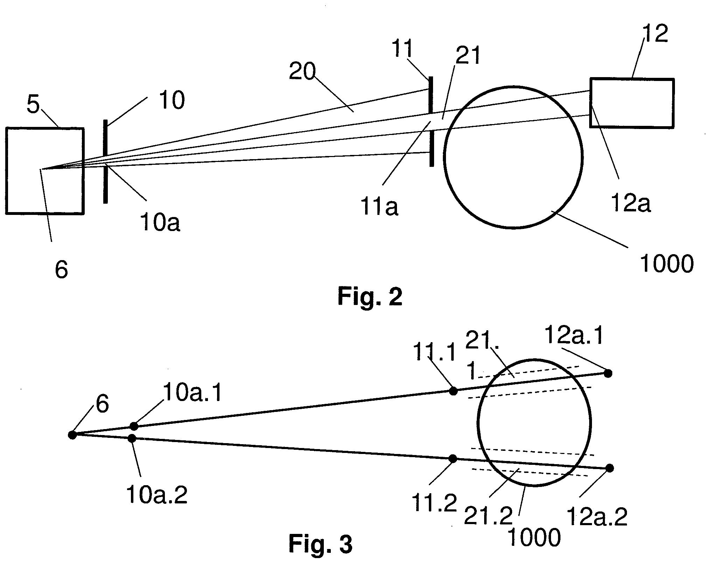

Fig. 2 is a diagram that shows a top view illustrating alignment of a center of a primary collimator, a center of a secondary collimator and a center of a sensor during a cephalometric scan. As shown on Fig. 2, a correct alignment of a center 10a of the aperture of the primary collimator 10, a center 11a of the aperture of the secondary collimator 11 and a center 12a of the imaging detector 12 is required at any step of the imaging scan. The X-ray beam emitted by the X-ray source 5 from the focal point 6 is first shaped by the primary collimator 10 into a slit beam 20. The slit beam radiates the collimator 11 that shapes the beam 20 into a slit beam 21 (e.g., narrower slit beam). The slit beam 21 radiates a thin part of the patient's head positioned on the patient support 14 behind the collimator 11 and impinges the imaging detector 12.

In order to image the whole object, namely the entire patient's head, a scan (e.g., cephalometric scan) is performed. Only a portion of the skull of the patient 1000 is radiated at each position of the ensemble including the primary collimator 10, the secondary collimator 11 and the imaging detector 12. To scan the ensemble of the skull, the center of the aperture of the collimators as well as the center of the imaging device must be translated (e.g., horizontally) in a synchronized manner. During one exemplary scan, the lateral blades or shutters of the primary collimator 10 can be horizontally displaced, while the secondary collimator 11 can be translated.

Fig. 3 is a diagram that shows a top view illustrating the translation of a center of a primary collimator, a center of a secondary collimator and a center of a detector during a cephalometric scan. At each scan step .1 (respectively .2) the location 10a.1 (respectively 10a.2) of the center 10a of the primary collimator 10, the location 11a.1 (respectively 11 a.2) of the center 11a of the secondary collimator 11 and the location 12a.1 (respectively 12a.2) of the center 12a of the detecting area of the imaging detector 12 are preferably or required to be aligned. In a preferred exemplary embodiment, the secondary collimator and the lining detector are displaced in translation along two parallel rails (not represented in Fig. 3) at velocities in a constant ratio, equal to the ratio d₆₋₁₁/d₆₋₁₂, of the distances from the focal spot 6 to the secondary collimator 11 and the distance from the focal spot 6 to the detector 12 respectively. At each position, the slit x-ray beam (e.g., slit beam 21) radiates a part of the patient's head. At the end of the cephalometric scanning process, the whole patient's head 1000 has been radiated.

Motion of various objects can be synchronized by a controller (e.g., microprocessor) of the cephalometric imaging device or using a mechanism. The synchronization of the secondary collimator 11 and the detector 12 can be done mechanically or electromechanically. The displacement of the center 10a of the aperture of the primary collimator 10 is synchronized with the motion of the secondary collimator 11 and imaging detector 12 using one or more microprocessors/computers of the dental imaging device.

As an alternative exemplary embodiment, instead of displacing the aperture of the primary collimator 10 relative to the X-ray source 5, it is possible to displace the X-ray source 5 during the scan imaging. For example, the source can be rotated.

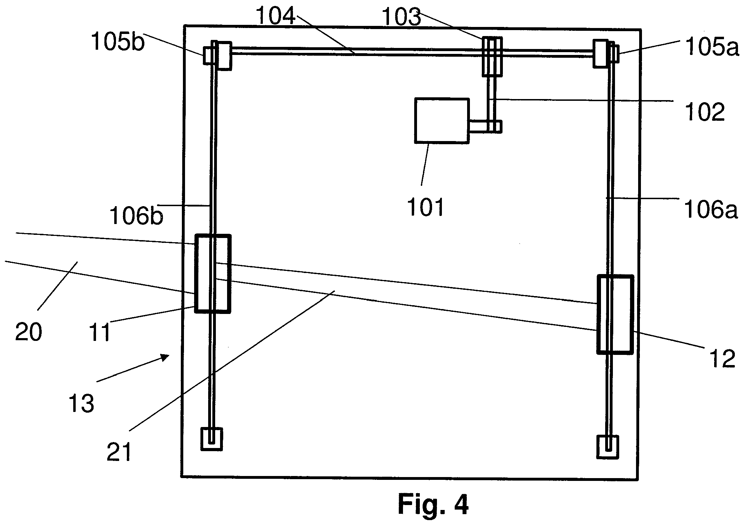

Fig. 4 illustrates a non-limitative example of a mechanism to synchronize the motion of the secondary collimator 11 and the imaging detector 12. As shown in Fig. 4, a motor 101 drives a belt 102. The belt 102 cooperates with a gear 103 that makes a shaft 104 rotate. Coupled to the shaft 104, two gears of different size 105a and 105b cooperate with belts 106a and 106b respectively. The belt 106b, associated with the smallest gear 105b, cooperates with the secondary collimator 11 while the belt 106a, associated with the biggest gear 105a, cooperates with the imaging detector 12. In one embodiment, the ratio of the speeds of the secondary collimator 11 and the imaging device 12 is equal to the ratio of the radius of gears 105a and 105b. The ratio of the speed of motion of the secondary collimator and the imaging detector can then be appropriately or definitely set by selected dimensioning of the gears 105a and 105b.

However, because of dimensions and/or clearances between the belts 106a and 106b with the gears 105a and 105b respectively, the secondary collimator 11 and the imaging detector 12 do not remain at the desired or requested relative position during the entire scan.

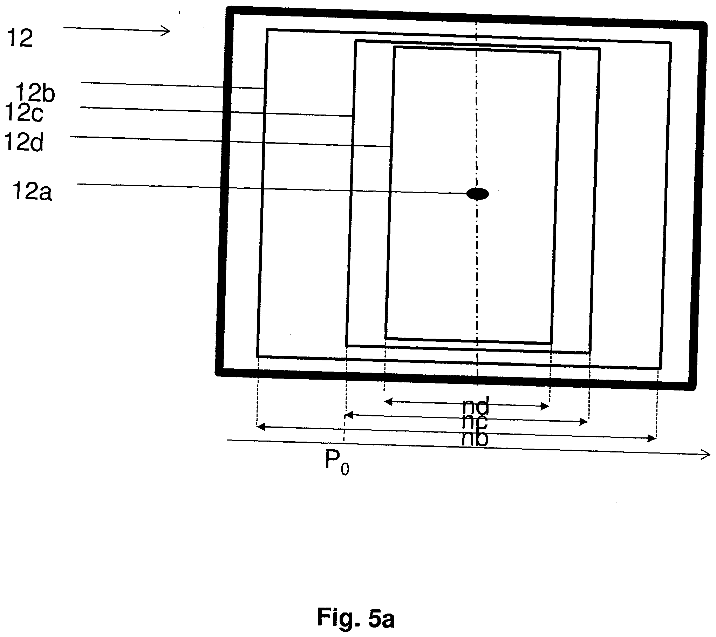

Fig. 5a illustrates a desired or ideal irradiating situation corresponding to the case where the secondary collimator 11 and the imaging detector 12 are correctly or perfectly aligned. The imaging detector 12 includes an active area 12b with a number nb of pixels in the width dimension. After the slit beam 21 has passed the secondary collimator 11, the slit beam 21 radiates a radiated area 12c having a number of nc pixels in width centered on the center 12a of the active area 12b. In the vicinity of the edges of the active area 12b, the signal may be slightly degraded or spoilt, for example, by the edges effects. Usually, the parts of the X-ray beam that passes at the vicinity of the edges of the aperture of the secondary collimator 11 are partly obstructed and scattered. Consequently, in cephalometirc scan imaging is preferable to crop the signal given by the pixels at the periphery of the radiated area 12c in order to keep for image reconstruction only an imaging area 12d of smaller width (than the radiated area 12c) centered on the active area 12b. In the case of a perfect alignment of the secondary collimator and the imaging device as shown on Fig. 5a, the cropped area 12d is also centered on the radiated area 12c. The pixels of the active area out of the radiated area 12c provide a white signal, for example corresponding to non radiated pixels, and need to be or are preferably cropped as well. If a width of nd pixels is set for the imaging (cropped) area 12d, a number of pixels equal to (nb-nd)/2 on each border of the active area 12b of the imaging detector 12 can be cropped.

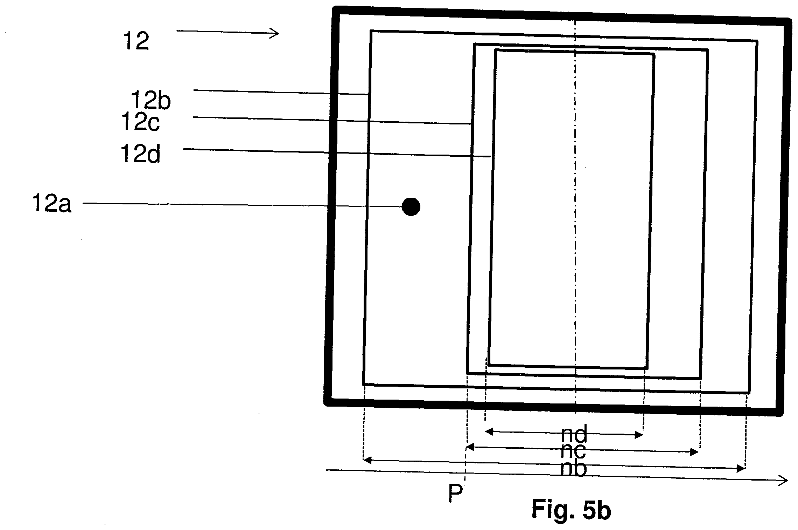

If the position and extent of this crop is set automatically on each of the frames acquired at each position of the secondary collimator 11 and the imaging device simply by cropping symmetrically a fixed number of pixels on each border of the active area, then a misalignment (e.g., appearing and evolving during the scan) of the secondary collimator 11 and the imaging detector 12 leads to an incorrect designation of the pixels to read from the imaging detector 12. In this case, as illustrated on Fig. 5b, while the imaging area (or cropped area) 12d is centered on the active area 12b, the radiated area 12c, which is offset relative to the active area 12b, is also offset relative to the imaging or cropped area 12d. Some data corresponding to the part of the X-ray beam that passed at the vicinity of the edges of the secondary collimator 11 are used in the image reconstruction, leading to a decrease of the quality of the reconstructed area.

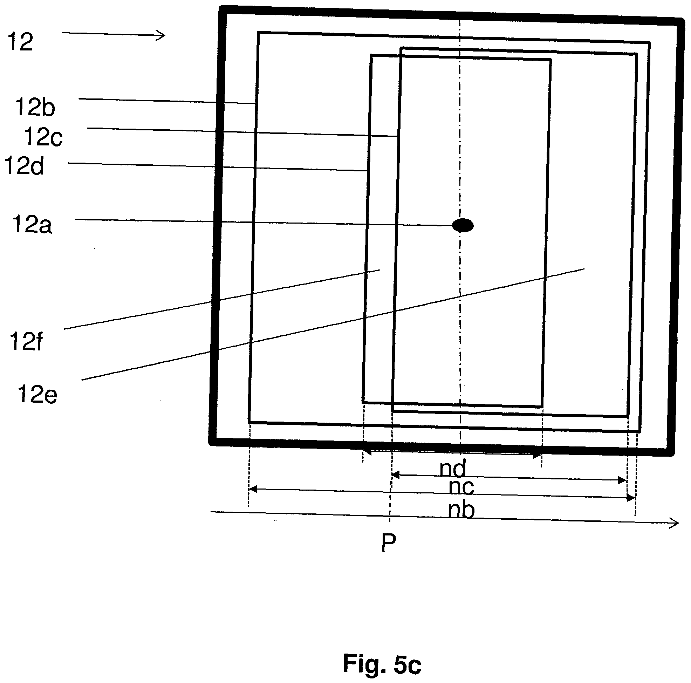

Fig. 5c illustrates an even worse situation. The actual radiated area 12c of the imaging detector 12 extends at least partially out of the cropped area 12d. The position P of the left edge of the radiated area 12c is then at a position P such as P-P₀>(nc-nd)/2, where P₀ is the position of the left edge of the radiated area 12c in the ideal aligned case of Fig. 5a. As shown in Fig. 5c, there is an area 12e inside the radiated area 12c where relevant information about the patient is cropped and an area 12f where some non radiated pixels are read and information thereof is used for image reconstruction, which can lead to errors such as a whitening of the image.

Thus, in the related art, due to the clearance in the mechanical interaction between pieces of the coupling mechanism, the relative position of the secondary collimator and the slit imaging device may differ from the expected position. Consequently, the position of the center of the aperture of the two collimators and the center of the slit imaging device are misaligned, resulting in a non centered radiation of the slit imaging device. During the stitching, the signal provided by non illuminated pixels are summated with the rest of the frames, leading to horizontal and vertical white lines on the final anatomical image of the patient. These defaults may complicate diagnostic actions by the practitioner. It may be costly and complicated to improve the mechanics of the coupling mechanism between the secondary collimator and the slit imaging device. While such systems may have achieved certain degrees of success in their particular applications, there is consequently a need for improving the quality of the cephalometric image. In particular, there is a need for a computer implemented method for improving the quality of the cephalometric image (e.g., cephalometric scan imaging).

Exemplary method and/or apparatus embodiments according to the application can to implement a dynamic cropping for the cropped area 12d on the active area 12b for each frame obtained during a scan imaging. Certain exemplary embodiments according to the application can implement a dynamic cropping process by dynamic detection of the position P of an edge of the radiated area 12c on the active area 12b to dynamically set the position of the cropped area 12d on each frame. In one exemplary embodiment, the edge may be a lateral edge that extends in a direction transverse to the scanning direction. In another exemplary embodiment, the transverse edge is orthogonal to the scanning direction.

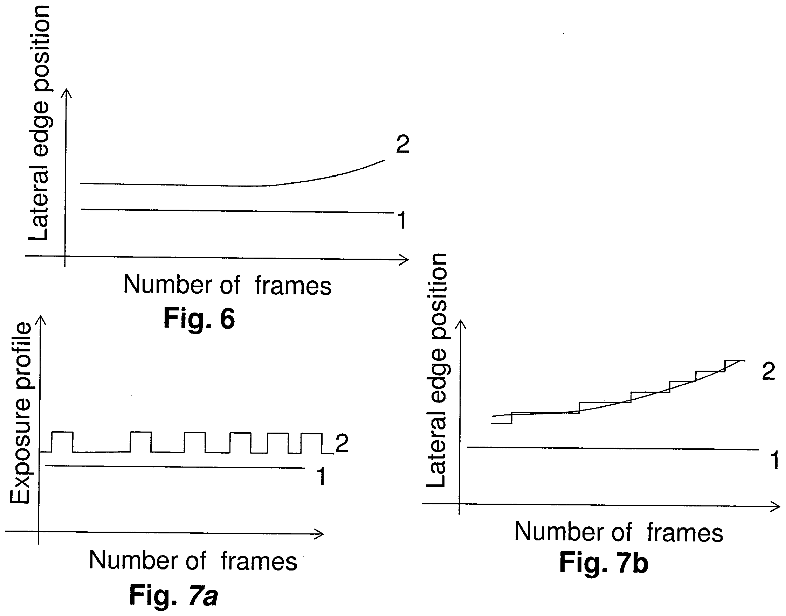

During a cephalometric scan, the position of an edge (for example the left edge) of the radiated area on the active area of the imaging device is intended to be or ordered at a set and constant position during the whole scan (edge position curve 1 on Fig. 6). As described herein, the clearance between the mechanical elements of Fig. 4, in actuality, causes a slight progressive relative displacement of the secondary collimator and the imaging device relative to their ordered position during the scan (edge position curve 2 on Fig. 6). The edge position curve is of the type sketched by edge position curve 2 on Fig. 6 and represents the position of an edge of the irradiated area on a frame as a function of the position of the frame amongst the set of frames acquired during the scan.

The edge position curve may correspond to the position of either the left or right edge position. Further, instead of using an edge position curve, exemplary embodiments of the application can also use a center position curve giving the position of the center of the irradiated area on each frame, in the exact middle of the position of both detected edges.

As used herein, an exposure profile is the overlapping of the frames taken during a scan (or scan imaging) as a function of the position of the frames during the scan. The exposure profile defines the positions of the trajectory at which the frames are captured.

A preset trajectory, namely the position at any time of the scan of the secondary collimator and detector imaging, is stored on (e.g., at the microprocessor of) the imaging device. The microprocessor can give instructions or control to the motor 101 for the displacement of the collimator, secondary collimator and imaging device. During the displacement, frames are acquired by the imaging detector 12 at a constant frame rate, typically 300frames/second. Additionally, a device can give feedback of the actual position of the imaging detector that may be different from the ordered position and gives feedback to the microprocessor. In one embodiment, the motor is a stepping motor and the actual stepping position information of the motor is sent to the microprocessor of the imaging device. Based on the actual positions of the collimator and imaging device, a non corrected exposure profile, namely without the use of exemplary method and/or apparatus embodiments described herein, can be defined. This non corrected exposure profile defines the overlap between successive frames by taking into account the actual position of the secondary collimator and imaging devices, positioned by motor 101, but does not take into account of the relative misalignment of these two elements that can be caused by coupling mechanism (e.g., the clearance between the belts 106 a and 106b and the gears 105 a and b (Fig. 4). In other words, the non corrected exposure profile considers that the irradiated area 12c is centered on the active area 12b throughout the entire scan.

It will be apparent to the man skilled in the art that all the exposure profiles (non corrected and actual exposure profiles) are noisy because they are calculated on feedback of imaging device's position, though the exposure profiles are represented as sharp curves. As an alternative, it is possible to calculate the exposure profile only on the basis of the trajectory that is to say without carrying on the position adjustment by a feedback described above. In that case, the real position of the imaging device and the sensor is not taken into account in the determination of the exposure profile, but only the ordered position.

As described herein, in a linear cephalometric scan, the trajectory is a translation at constant speed of the secondary collimator 11 and imaging device 12. The non corrected exposure profile (curve 1 on Fig. 7a) of a standard linear cephalometric scan corresponds to an almost constant overlapping of frames, the successive frames being taken almost at regularly spaced positions of the secondary collimator 11 and imaging device 12 along the trajectory, notwithstanding the feedback adjustment process.

The non corrected exposure profile 1 (respectively 2) of the Fig. 7a then corresponds to the edge position curve 1 (respectively 2) of Fig. 7b. The offset by one pixel of the edge position in one frame corresponds to an increase (or decrease) of one pixels in the overlap of this frame with the previous frame.

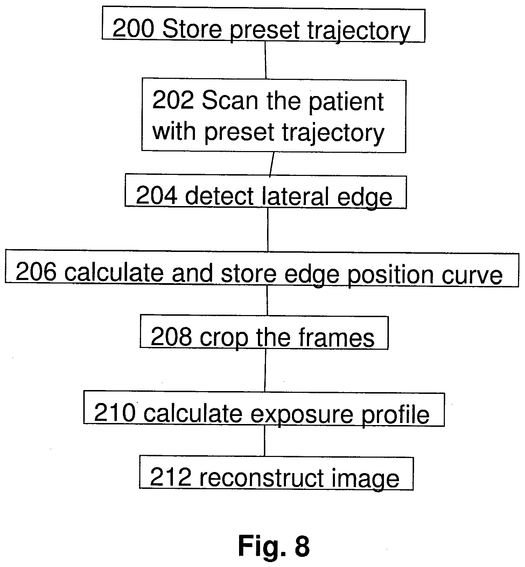

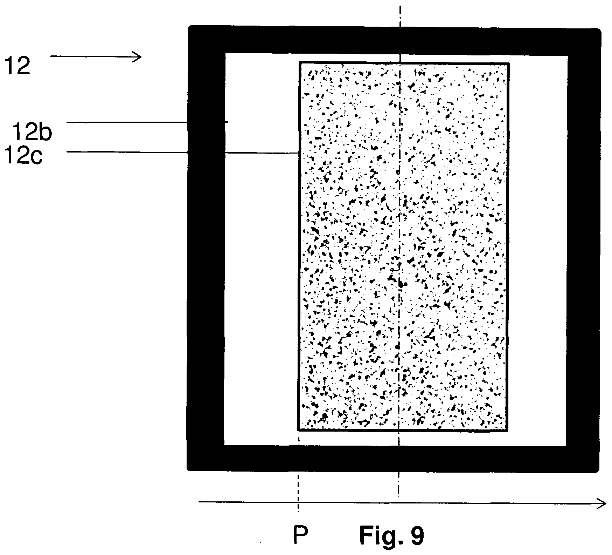

Fig. 8 is a flowchart that shows a first exemplary cephalometric imaging method embodiment according to the application. As shown in Fig. 8, In step 200, a trajectory is stored in the processor of the cephalometric imaging device. The corresponding non corrected exposure profile, that is to say with the feedback adjustment, is the exposure profile represented on the curve 1 of the Fig. 7a. A patient 1000 is positioned on the patient support 14. In step 202, the cephalometric imaging device then scans the head of a patient positioned on the patient support 14. The scan is carried out in accordance with the trajectory stored on step 200. A plurality of frames is then acquired at constant frame rate. Then, in step 204, a conventional imaging data treatment known in the art detects the position of at least one of the lateral edges of the irradiated area on at least one frame. The frames each contains a white area corresponding to the non-radiated part of the imaging device and an area with various levels of grey corresponding to the impingement of the x-ray beam on the imaging device (e.g., see Fig. 9). Fig. 9 is a diagram that shows an exemplary frame imaged during a scan of a patient. These x-rays may have passed through the patient's anatomy and have been more or less absorbed, then giving rise to a signal corresponding to various grey levels. The x-ray may also have directly impinged the imaging device at the very beginning or at the very end of the scan when the imaging device and secondary collimator are at their start or end positions of their trajectory. In this case, the radiated area is represented as a black area.

In step 206, the positions P of the at least one lateral edge of the radiated area of at least some of the frames acquired at step 202, are calculated and stored. In a preferred exemplary embodiment, the detection of at least one edge of the irradiated area (step 204) is performed for all the frames. Alternatively, it is possible in the implementation of some exemplary method embodiments to detect at least one edge on only some of the frames. In that case, the step 206 can include a first substep for determining a first set of positions of the edge for the frames for which step 204 is carried out and a second substep for interpolating the positions of the edge for the other frames using the first set of positions. In still another exemplary method embodiment, it is possible to detect at least one edge on only one single frame. In that case, an offset can be detected between the ordered positions and actual positions of the radiated area on the active area. The positions P are stored, for example, in the microprocessor.

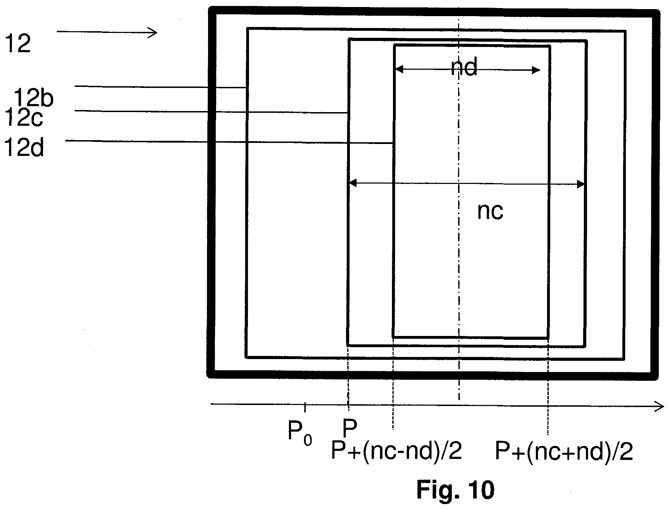

In step 208, the frames are cropped using the positions calculated and stored on step 206. Fig. 10 is a diagram that shows an exemplary dynamic crop of a frame, the cropped area being defined by an actual position of a lateral edge of the radiated area of an imaging detector. As shown in Fig. 10, the width (in number of pixels) of the irradiated area 12c is nc and the position of the left edge of the active area 12c on the active area 12d is P. The cropped area 12d having a width of nd pixels must be centered on the irradiated area 12c. Then the crop operation consists in conserving only the information relative to pixels located between the positions P+(nc-nd)/2 and P+(nc+nd)/2.

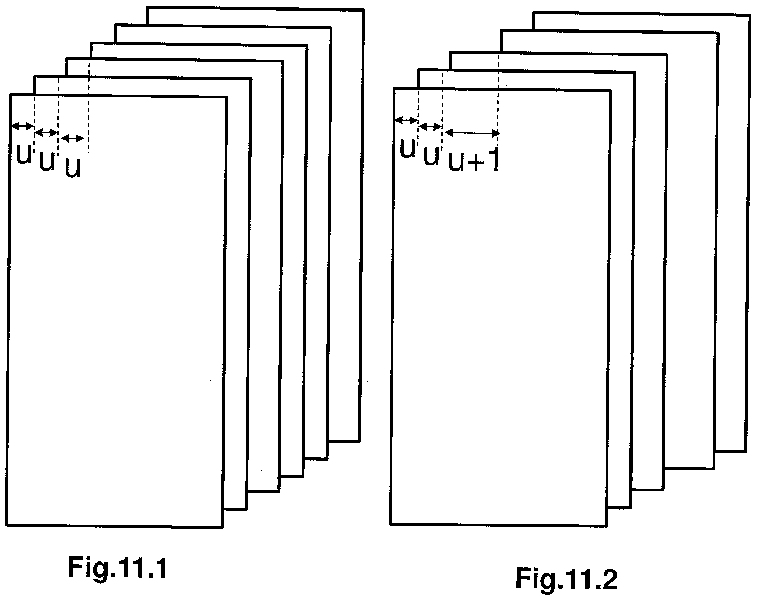

In step 210, the actual exposure profile (curve 2 on Fig. 7.b), different from the non corrected exposure profile coming after feedback adjustment, from the scan of the patient, is calculated on the basis of the positions measured on step 204 and calculated and stored on step 206 and on the non corrected exposure profile. Then, in step 212 the image is reconstructed using the actual exposure profile (Fig. 11.2). Fig. 11.1 represents the reconstruction of the final image according to the prior art, using the non corrected exposure profile, that is to say without correction of the offset of the irradiated area. Fig 11.2 represents the correct reconstruction taking into account the offset, that is to say by applying the actual exposure profile (Fig. 11.2) according to exemplary embodiments of the application.

Instead of detecting at least one edge of the irradiated area on the frames acquired along the scan of a patient, selected exemplary embodiments for dynamic cropping for an irradiated area on the an active area of an imaging device for each frame obtained during a scan imaging carry out a blank scan at the time of the installation of the device in the dental site. In this blank scan, the non radiated area of the frame is white and the irradiated area is black and not grey as it is the case for frames acquired during the scan of a patient. The detection of the edges is facilitated because the contrast between radiated and non-radiated is stronger in the frames acquired during a blank scan than in the frames acquired during the scan of a patient.

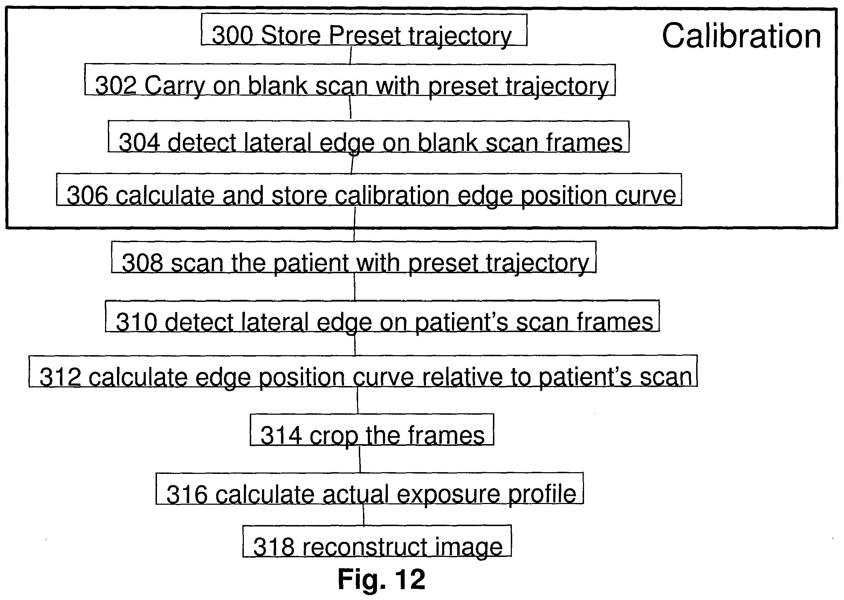

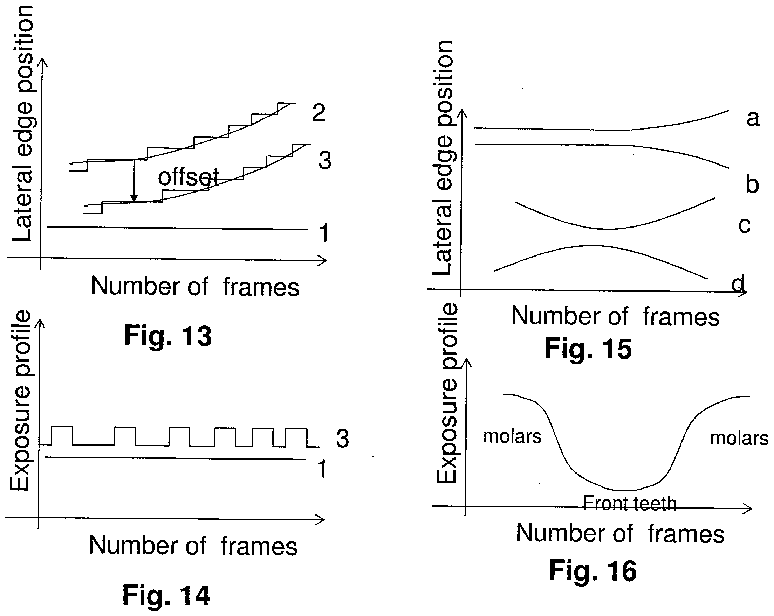

Fig. 12 is a flowchart that shows a second exemplary cephalometric imaging method embodiment according to the application. As shown in Fig. 12, in step 300, a preset trajectory is stored in the processor of the cephalometric imaging device corresponding to a (feedback adjusted) non corrected exposure profile (curve 1 of the Fig. 14). In step 302, the imaging device then carries out a blank scan, that is to say a scan without a patient positioned on the patient support. The scan is carried out in accordance with the stored trajectory. A plurality of frames is then acquired. In step 304, a conventional imaging data treatment known in the art can detect the position of at least one of the edges (e.g., lateral) of the irradiated area on at least one frame of the blank scan.

In step 306, the positions P of the at least one lateral edge of the radiated area of at least some of the frames acquired at step 302, are calculated and stored. Again, in one preferred exemplary embodiment, the detection of at least one edge of the irradiated area (step 304) is performed for all the frames. Alternatively, it is possible to detect at least one edge on only some of the frames. In that case, the step 306 can include first determining a first set of positions of the edge for the frames for which step 304 was carried out and second interpolating positions of the edge for the other frames using the first set of positions. In still another exemplary embodiment, it is possible to detect at least one edge on only one single frame. In that case, an offset can be detected between the ordered positions and actual positions of the radiated area on the active area. The positions P can be stored at the cephalometric imaging device, for example, in the microprocessor. The positions P in an ideal situation are represented on the edge position curve 1 of Fig. 13, the actual positions P obtained from the blank scan are represented on the calibration edge position curve 2 of Fig. 13.

According to embodiments of the application, steps 300 to 306 can corresponds to a calibration step that may be carried out during the installation of the x-ray apparatus, for example, at the dental site. This calibration step may not be reproduced for each patient before the steps described below that are specifically directed to the scan of each patient. Further, the calibration step, steps 300 to 306 may be carried out one single time for a plurality of patients, while the steps 308 to 318 are carried out for each patient and take the benefit of the results of the calibration steps 300 to 306. In an alternative embodiment, calibration step according to steps 300 to 306 can also be carried out before each patient's scan. In another alternative embodiment, calibration step according to steps 300 to 306 can also be carried out periodically in time or upon a detected error condition.

In step 308, the patient is scanned using the preset trajectory (leading to the non corrected exposure profile represented on curve 1 on Fig. 14). In a successive step 310, at least one lateral edge of the radiated area 12c is determined on at least one frame acquired during step 306. It has been observed that the edge position curve may be offset from one scan to the other but keep the same general profile. Consequently, by comparing the calibration edge position curve for the positions P of the lateral edge of the irradiated area 12c on a frame obtained at step 302 during the blank scan and the edge position curve for the positions P of the lateral edge of the irradiated area 12c on a frame obtained at step 308 during the scan of a patient at the same moment of the scan, it is possible to calculate the magnitude of the offset. The edge position curve (curve 3 on Fig. 13) can then be calculated (step 312) on the basis of the determined offset and the calibration edge position curve of the blank scan.

On exemplary way to calculate the edge position curve 3 (Fig. 13) relative to the scan of the patient is to interpolate the calibration edge position curve 2 relative to the blank scan by a polynomial function of the first or second degree. The same polynomial function is simply offset to obtain the edge position curve 3.

Once the edge position curve 3 of the irradiated areas relative to the patient's scan is known or determined, it is then possible to crop the frames, as described in the exemplary method shown in Fig. 8. In the step 314, the frames are cropped using the calculated positions of step 312. As shown in Fig. 10, the width (in number of pixels) of the irradiated area 12c is nc and the position of the left edge of the active area 12c on the active area 12d is P. A cropped area 12d having a width of nd pixels must be centered on the irradiated area 12c. Then, the crop operation consists in conserving only the information relative to pixels located between the positions P+(nc-nd)/2 and P+(nc+nd)/2. This exemplary method can also be implemented with a variable width nd of the cropped area nd along the scan. Then a preset nd profile is stored in the microprocessor of the cephalometric imaging device and the variable nd value is used in the equations mentioned above.

In step 316, the actual exposure profile (Curve 3 on Fig. 14), different from the non corrected exposure profile (coming from the preset trajectory) used for the scan of the patient (Curve 1 on Fig. 14), is calculated on the basis of the edge position curve calculated on Step 314 (curve 3 on Fig. 13) and the non corrected exposure profile of the scan of the patient. Then in step 318, the image is reconstructed using the actual exposure profile (e.g., Fig. 11.2).

It must be noted that an edge position curve does not necessarily have the profile represented on Fig. 13. From a general standpoint, exemplary embodiments according to application can include different edge position relationships. For example, the edge position curve may be an increasing function of the position of the frames on the final image (curve a on Fig. 15). It may also be a decreasing function (curve b), or even have a minimum value (curve c) or a maximum value (curve d). Other edge position relationships can also be envisioned. In any case, it is possible to interpolate the edge position curve, for example, by a polynomial function, preferably of the first or second degree.

As shown in Fig. 1, the gantry 3 of the imaging device 1 can include a second imaging detector 7 that may be a panoramic imaging detector. This imaging device is an elongated imaging detector of the same type as the cephalometric imaging device. In some exemplary embodiments, the cephalometric imaging detector 12 and the panoramic imaging detector 7 are one single imaging device that can be plugged and unplugged from a first position at the end of the cephalometric arm 9 to a second position at one extremity of the gantry 3 depending on the type of imaging that is required. The x-ray source 5 and the panoramic imaging detector 7 are facing each other on a fixed relative position with a patient positioned on the patient's holder 8 in-between.

During a panoramic scan, the gantry 3 follows a preset trajectory composed of selective translation and selective rotation so that the slit x-ray beam generated by the x-ray source 5 and shaped by the primary collimator 10 radiates sequentially the whole dental arch. The actual position of the gantry (XY position and angular position) is sensed by dedicated sensors and, like in the cephalometric linear scan, the exposure profile, that defines the overlapping of frames along the trajectory of the source and imaging device, is calculated using a feedback signals sent all along the preset trajectory. The exposure profile of a panoramic scan is U-shaped or V-shaped (see Fig. 16). The panoramic exposure or scan starts with the radiation of molars on one side of the jaw with an overlap of the frames (or density of frames) defined by the trajectory and adjusted by the feedback signal. The scan continues with the radiation of the front teeth, for which region the overlap of frames (or the density of frames) is increased compared to the overlap of frames relative to the region of the molars. The exposure profile then reaches a minimum at the position of the front teeth. The panoramic scan finishes with the radiation of the molars on the opposite side of the jaw with again an overlap of frames (or density of frames) that is smaller than for the radiation of the front teeth.

The primary collimator 10 may have a variable aperture. The variable aperture of the primary collimator 10 can be implemented for example with a blade or shutter collimator or the like. The width of the aperture of the primary collimator 10 can be varied during the panoramic scan to vary the width of the focal trough, which is the area of sharpness, of the panoramic image. Then, a width profile defines the width of the aperture of the collimator 10 at any position of the X-ray source and imaging device along their trajectory during the panoramic scan. If one of the lateral blades or shutters of the primary collimator 10 does not position at the preset location, then the irradiated area of the imaging device is not exactly centered on the imaging device and may be cropped inadequately or degrade the reconstructed panoramic image.

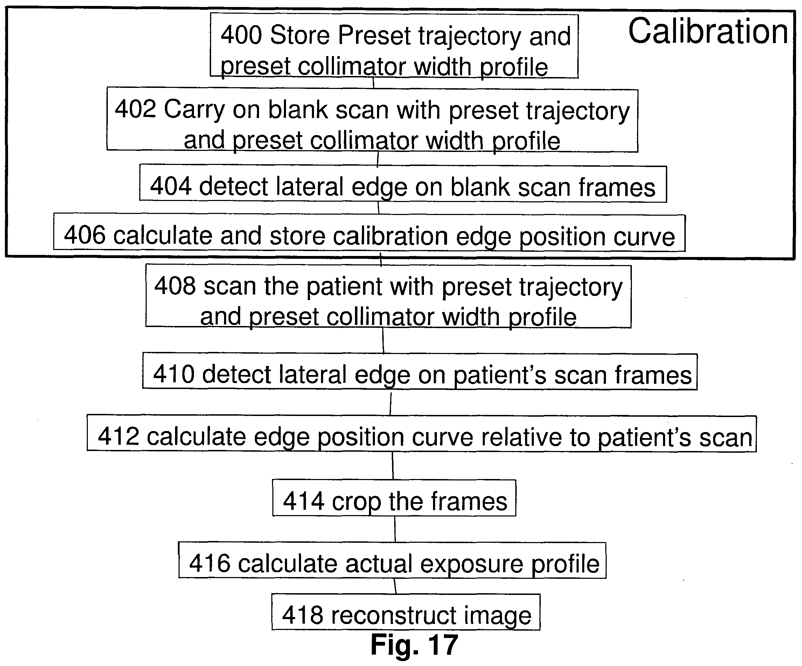

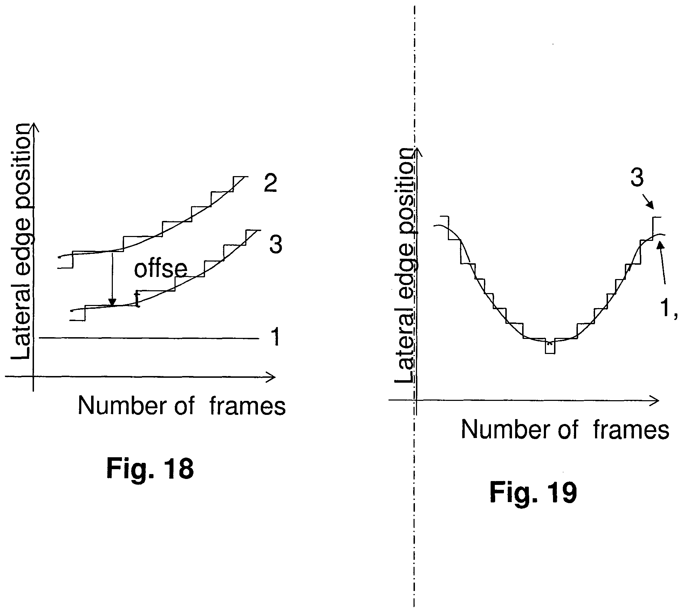

Fig. 17 is a flowchart that shows an exemplary panoramic imaging method embodiment according to the application. As shown in Fig. 17, in step 400, a preset trajectory for the gantry 3 (giving raise after feedback adjustment to the non corrected exposure profile illustrated on curve 1 of the Fig. 19) and a preset width profile (e.g., variable) for the collimator 10 are stored in the panoramic imaging device. In one embodiment, the processor in the panoramic imaging device can store a plurality of preset selectable trajectories. In step 402, the imaging device then carries out a blank scan being a scan without a patient positioned on the patient support. The blank scan is carried out in accordance with the stored trajectory and the stored width profile of the collimator. A plurality of frames is then acquired, preferably at constant frame rate during the blank scan. In step 404, a conventional imaging data treatment known in the art can detect the position of at least one edge (e.g., lateral, top, corners) of the irradiated area on at least one frame.

In step 406, the positions P of the at least one lateral edge of the radiated area of at least some of the frames acquired at step 402, are calculated and stored. In one exemplary embodiment, the detection of at least one edge of the irradiated area (step 404) is performed for all the frames from the panoramic blank scan. Alternatively, it is possible to detect at least one edge on only some of the frames. In that case, the step 406 includes determining a first set of positions of the at least one edge for the frames for which step 404 was carried out and then interpolating the positions of the at least one edge for the other frames (remaining frames) using the first set of positions. In still another exemplary embodiment, it is possible to detect at least one edge on only one single frame. In that case, an offset can be detected between the ordered position and actual positions of the radiated area on the active area. The edge position curves for the positions P are preferably stored in the microprocessor. The positions P in an ideal situation are represented on the curve 1 of Fig. 18 and the calibration positions P obtained from the blank scan are represented on the curve 2 of Fig. 18.

The succession of steps 300 to 306 can correspond to a calibration step that may be carried out during the installation of the x-ray apparatus at the dental site. This calibration step may not be reproduced for each patient before the steps described below that are specifically directed to the scan of each patient. Alternatively, the calibration steps 300 to 306 may be carried out one single time for a plurality of patients, while the steps 308 to 318 are carried out for each patient and take the benefit of the results of the calibration steps 300 to 306. In an alternative exemplary embodiment, calibration step according to steps 300 to 306 can also be carried out before each patient's scan.

In step 408, the patient is scanned using the preset trajectory and preset width profile stored at step 400. In successive step 410, at least one lateral edge of the radiated area 12c is determined on at least one frame acquired during step 406. It has been observed by the inventors that the edge position curve is offset from one scan to the subsequent one but keeps the same general shape. Consequently, by comparing the position P of the lateral edge of the irradiated area 12c on a frame obtained at step 402 during the blank scan (calibration edge position curve) and the position P of the lateral edge of the irradiated area 12c on a frame obtained at step 408 during the scan of a patient at a corresponding point or the same moment of the scan, it is possible to calculate the magnitude of the offset. In step 412, the new edge position curve (curve 3 on Fig. 18) can then be calculated on the basis of the determined offset and the calibration edge position curve of the blank scan (curve 2).

The edge position curve (curve 3 on Fig. 18) being known, it is then possible to crop the frames, as described in the exemplary method shown in Fig. 8. In step 414, the frames are cropped using the edge position curve obtained at step 412 (curve 3 on Fig. 18).

As shown in Fig. 10, the width (in number of pixels) of the irradiated area 12c is nc for a given frame and the position of the left edge of the active area 12c on the active area 12d is P. A cropped area 12d having a width of nd pixels for a given frame must be centered on the irradiated area 12c. Then the crop operation consists in conserving only the information relative to pixels located between the positions P+(nc-nd)/2 and P+(nc+nd)/2. It must be noticed that, as the width of the collimator varies during the scan and is defined by a width profile, the width nc of the radiated area 12c varies as well. It would be consistent that the width nd of the cropped area 12d varies as a function of the width nc. In one exemplary embodiment, the width nd is defined by a profile as well.

In step 416, the actual exposure profile (curve 3 on Fig. 19), different from the non corrected exposure profile obtained from the preset trajectory used for the scan of the patient (curve 1 on Fig. 19), is calculated on the basis of the edge position curve calculated on step 414 (curve 3 on Fig. 18) and on the non corrected exposure profile. Then, in step 418, the image is reconstructed using the actual exposure profile (see Fig 11.2). In Fig. 17, the scan can be a panoramic scan of the dental arch.

The invention has been described in detail with particular reference to a presently preferred embodiment, but it will be understood that variations and modifications can be effected within the scope of the invention. In addition, while a particular feature of the invention can have been disclosed with respect to one of several implementations, such feature can be combined with one or more other features of the other implementations as can be desired and advantageous for any given or particular function. Further, "exemplary" indicates the description is used as an example, rather than implying that it is an ideal. The presently disclosed embodiments are therefore considered in all respects to be illustrative and not restrictive. The scope of the invention is indicated by the appended claims.

## Claims

1. A method of cephalometric X-ray imaging of a region of interest of a patient with an X-ray apparatus (1) comprising:
an X-ray source (5) to emit an X-ray beam;
at least one collimator (10, 11) to shape the X-ray beam;
an X-ray line detector (12) including a plurality of detection elements arrayed in the width direction and the length direction to receive the shaped X-ray beam;
a storing unit configured to store at least one trajectory for the collimator and for at least one of said X-ray source (5) and said X-ray line detector (12) for a scan imaging;
a manipulator to displace an aperture of said at least one collimator (10, 11) and at least one of said X-ray source (5) and said X-ray line detector (12) along said at least one trajectory; and
an image reconstruction unit;
the method comprising:
performing (202; 308; 408) a first scan imaging of a region of interest of a patient with the aperture of the at least one collimator (10, 11) and the at least one of said X-ray source (5) and said X-ray line detector (12) following a first scan trajectory of said at least one stored trajectory;
collecting (308; 408) a plurality of frames from an exposure profile of said first scan imaging of the region of interest of said patient, where the exposure profile is the overlapping of the plurality of frames taken during first scan imaging as a function of the position of the plurality of frames during the first scan imaging;
determining (204; 310; 410) a location of at least one edge of an irradiated area on at least one frame of the region of interest of said patient from said plurality of frames from said first scan imaging;
establishing (206; 312; 412) an edge position curve relative to the first scan imaging of the region of interest of said patient, where the edge position curve represents a position of an edge of the irradiated area on a frame as a function of the position of the frame;
cropping (208; 314; 414) selected frames of said plurality of frames from said first scan imaging on the basis of said edge position curve;
**characterized in that** the method further comprising
calculating (210; 316; 416) an actual exposure profile based on the edge position curve and the exposure profile; and
reconstructing (212; 318; 418) the region of interest of said patient by combining said cropped selected frames using said actual exposure profile.

2. The method according to claim 1, further comprising calibrating the first scan imaging of the X-ray apparatus by:
performing (302; 402) a blank scan imaging without the patient with the aperture of the at least one collimator (10, 11) having a preset width profile and at least one of said X-ray source (5) and said X-ray line detector (12) following said first scan trajectory;
collecting (302; 402) a plurality of frames from the blank scan imaging;
determining (304; 404) the location of at least one edge of the irradiated area on at least one frame of the plurality of frames from the blank scan imaging; and
establishing (306; 406) a calibration edge position curve relative to the blank scan imaging;
and wherein the edge position curve relative to the first scan imaging of the region of interest of said patient is established on the basis of the calibration edge position curve and said location of at least one edge of an irradiated area on the at least one frame of the region of interest of said patient.

3. The method according to claim 2 wherein the calibrating the first scan imaging of the X-ray apparatus (1) is carried out at the time of the installation of said X-ray apparatus (1) at the dental site or before the first scan imaging of the region of interest of said patient.

4. The method according to claim 1 wherein said X-ray apparatus (1) is a cephalometric imaging apparatus.

5. The method according to claim 4 wherein the at least one collimator (10, 11) is on the side of the X-ray line detector.

6. The method according to claim 5 wherein the at least one collimator (10, 11) is a blade collimator, a shutter collimator, or a plate with a slit aperture provided therein.

7. The method according to claim 5 wherein a cephalometric collimator (10) and the X-ray line detector (12) slide along two parallel rails.

8. The method according to claim 7 wherein motions of the cephalometric collimator (10) and the X-ray line detector (12) are synchronized in an ordered constant speed ratio.

9. The method according to claim 4 wherein a second collimator (11) is positioned in front of the X-ray source (5) and wherein the motion the aperture of the second collimator (11) is synchronized with the motion of a cephalometric collimator (10) and the X-ray line detector (12).

10. The method according to claim 1 wherein the at least one edge extends in a direction transverse to the scanning direction.

11. The method according to claim 10 wherein the at least one edge extends in a direction orthogonal to the scanning direction, and wherein the source rotates during the first scan imaging.

12. The method according to claim 1 wherein the width of the cropped area varies amongst the frames.

13. The method according to claim 2 wherein the X-ray apparatus is a panoramic apparatus (7) with the source (5) and the X-ray line detector (12) positioned opposite to each other on both extremities of a rotating gantry (3), the at least one collimator (10, 11) being a variable collimator positioned in front of the X-ray source (5) and wherein a collimator width profile is stored in the storing unit.

14. The method according to claim 13 wherein the width of the cropped area varies amongst the frames, and wherein the width of the cropped area depends on the width of the irradiated area.

15. An X-ray apparatus (1) to image a region of interest of an imaging area of the apparatus (1) comprising:
an X-ray source (5) to emit an X-ray beam;
at least one collimator (10, 11) to shape the X-ray beam;
an x-ray line detector (12) including a plurality of detection elements arrayed in the width direction and the length direction and configured to receive the shaped X-ray beam;
a storing unit configured to store at least one trajectory for the collimator and for at least one of said X-ray source and said X-ray imaging device for a scan imaging;
a manipulator configured to displace an aperture of said at least one collimator (10, 11) and at least one of said X-ray source (5) and said X-ray line detector (12) along said at least one trajectory; and
an image reconstruction unit;
the apparatus (1) being configured to:
perform a first scan imaging of a region of interest of an imaging area with the aperture of the at least one collimator (10, 11) and the at least one of said X-ray source (5) and said X-ray line detector (12) following a first scan trajectory of said at least one stored trajectory;
collect a plurality of frames from an exposure profile of said first scan imaging of the region of interest of said patient, wherein the exposure profile is the overlapping of the plurality of frames taken during first scan imaging as a function of the position of the plurality of frames during the first scan imaging;
determine a location of at least one edge of an irradiated area on at least one frame of the region of interest of said imaging area from said plurality of frames from said first scan imaging;
establish an edge position curve relative to the first scan imaging of the region of interest of said patient, where the edge position curve represents a position of an edge of the irradiated area on a frame as a function of the position of the frame;
crop selected frames of said plurality of frames from said first scan imaging on the basis of said edge position curve; **characterized in that**
calculate an actual exposure profile based on the edge position curve and the exposure profile; and
reconstruct the region of interest of said imaging area by combining said cropped selected frames using said actual exposure profile.

## Patentansprüche

1. Verfahren zur kephalometrischen Röntgenaufnahme eines Bereichs von Interesse eines Patienten mit einer Röntgenvorrichtung (1), das Folgendes aufweist:
eine Röntgenquelle (5) zum Emittieren eines Röntgenstrahls;
wenigstens einen Kollimator (10, 11) zum Formen des Röntgenstrahls;
einen Röntgen-Zeilendetektor (12), der eine Vielzahl von Detektionselementen aufweist, die in der Breitenrichtung und der Längsrichtung angeordnet sind, um den geformten Röntgenstrahl zu empfangen;
eine Speichereinheit, die konfiguriert ist, um wenigstens eine Trajektorie für den Kollimator und für wenigstens eine der Röntgenquellen (5) und den Röntgen-Zeilendetektor (12) für eine Scanabbildung zu speichern;
einen Manipulator zum Verschieben einer Öffnung des wenigstens einen Kollimators (10, 11) und wenigstens die eine Röntgenquellen (5) und/oder des Röntgen-Zeilendetektors (12) entlang der wenigstens einen Trajektorie; und
eine Bildrekonstruktionseinheit;
wobei das Verfahren Folgendes aufweist:
Durchführen (202; 308; 408) einer ersten Scanabbildung eines Bereichs von Interesse eines Patienten mit der Öffnung des wenigstens einen Kollimators (10, 11) und wenigstens der einen Röntgenquelle (5) und/oder des Röntgen-Zeilendetektors (12), die einer ersten Scantrajektorie oder wenigstens einer gespeicherten Trajektorie folgen;
Sammeln (308; 408) einer Vielzahl von Einzelbildern aus einem Belichtungsprofil der ersten Scanabbildung des Bereichs von Interesse des Patienten, wobei das Belichtungsprofil die Überlappung der Vielzahl von Einzelbildern ist, die während der ersten Scanabbildung aufgenommen wurden, als Funktion der Position der Vielzahl von Einzelbildern während der ersten Scanabbildung;
Bestimmen (204; 310; 410) einer Position von wenigstens einer Kante eines bestrahlten Bereichs auf wenigstens einem Einzelbild des Bereichs von Interesse des Patienten aus der Vielzahl von Einzelbildern aus der ersten Scanabbildung;
Herstellen (206; 312; 412) einer Kantenpositionskurve in Bezug auf die erste Scanabbildung des Bereichs von Interesse des Patienten, wobei die Kantenpositionskurve eine Position einer Kante des bestrahlten Bereichs auf einem Einzelbild als Funktion der Position des Einzelbilds darstellt;
Zuschneiden (208; 314; 414) ausgewählter Einzelbilder der Vielzahl von Einzelbildern aus der ersten Scanabbildung auf der Grundlage der Kantenpositionskurve;
**dadurch gekennzeichnet, dass** das Verfahren ferner Folgendes aufweist:
Berechnen (210; 316; 416) eines tatsächlichen Belichtungsprofils basierend auf der Kantenpositionskurve und dem Belichtungsprofil; und
Rekonstruieren (212; 318; 418) des Bereichs von Interesse des Patienten durch Kombinieren der zugeschnittenen ausgewählten Einzelbilder unter Verwendung des tatsächlichen Belichtungsprofils.

2. Verfahren nach Anspruch 1, das ferner das Kalibrieren der ersten Scanabbildung der Röntgenvorrichtung durch Folgendes aufweist:
Durchführen (302; 402) einer Leerscanabbildung ohne den Patienten mit der Öffnung des wenigstens einen Kollimators (10, 11) mit einem vorbestimmten Breitenprofil und wenigstens einem aus der Röntgenquelle (5) und/oder den Röntgen-Zeilendetektor (12) der ersten Scantrajektorie folgend;
Sammeln (302; 402) einer Vielzahl von Einzelbildern aus der Leerscanabbildung;
Bestimmen (304; 404) der Position wenigstens einer Kante des bestrahlten Bereichs auf wenigstens einem Einzelbild der Vielzahl von Einzelbildern aus der Leerscanabbildung; und
Herstellen (306; 406) einer Kalibrierkantenpositionskurve bezüglich der Leerscanabbildung;
und wobei die Kantenpositionskurve bezüglich der ersten Scanabbildung des Bereichs von Interesse des Patienten auf der Grundlage der Kalibrierkantenpositionskurve und der Position wenigstens einer Kante eines bestrahlten Bereichs auf dem wenigstens einen Einzelbild des Bereichs von Interesse des Patienten hergestellt wird.

3. Verfahren nach Anspruch 2, wobei das Kalibrieren der ersten Scanabbildung der Röntgenvorrichtung (1) zum Zeitpunkt der Installation der Röntgenvorrichtung (1) an der Zahnstation oder vor der ersten Scanabbildung des Bereichs von Interesse des Patienten durchgeführt wird.

4. Verfahren nach Anspruch 1, wobei die Röntgenvorrichtung (1) eine kephalometrische Abbildungsvorrichtung ist.

5. Verfahren nach Anspruch 4, wobei sich der wenigstens eine Kollimator (10, 11) auf der Seite des Röntgen-Zeilendetektors befindet.

6. Verfahren nach Anspruch 5, wobei der wenigstens eine Kollimator (10, 11) ein Blattkollimator, ein Verschlusskollimator oder eine Platte mit einer darin vorgesehenen Schlitzöffnung ist.

7. Verfahren nach Anspruch 5, wobei ein kephalometrischer Kollimator (10) und der Röntgen-Zeilendetektor (12) entlang zweier paralleler Schienen gleiten.

8. Verfahren nach Anspruch 7, wobei Bewegungen des kephalometrischen Kollimators (10) und des Röntgen-Zeilendetektors (12) in einem geordneten konstanten Geschwindigkeitsverhältnis synchronisiert werden.

9. Verfahren nach Anspruch 4, wobei ein zweiter Kollimator (11) vor der Röntgenquelle (5) positioniert ist und wobei die Bewegung der Öffnung des zweiten Kollimators (11) mit der Bewegung eines kephalometrischen Kollimators (10) und des Röntgen-Zeilendetektors (12) synchronisiert wird.

10. Verfahren nach Anspruch 1, wobei sich die wenigstens eine Kante in einer Richtung quer zur Scanrichtung erstreckt.

11. Verfahren nach Anspruch 10, wobei sich die wenigstens eine Kante in einer Richtung orthogonal zur Scanrichtung erstreckt und wobei sich die Quelle während der ersten Scanabbildung dreht.

12. Verfahren nach Anspruch 1, wobei die Breite des zugeschnittenen Bereichs zwischen den Einzelbildern variiert.

13. Verfahren nach Anspruch 2, wobei die Röntgenvorrichtung eine Panoramavorrichtung (7) mit der Quelle (5) und dem Röntgen-Zeilendetektor (12) ist, die an beiden Enden eines Drehgerüsts (3) einander gegenüberliegend angeordnet sind, wobei der wenigstens eine Kollimator (10, 11) ein variabler Kollimator ist, der vor der Röntgenquelle (5) angeordnet ist, und wobei ein Kollimatorbreitenprofil in der Speichereinheit gespeichert ist.

14. Verfahren nach Anspruch 13, wobei die Breite des zugeschnittenen Bereichs zwischen den Einzelbildern variiert, und wobei die Breite des zugeschnittenen Bereichs von der Breite des bestrahlten Bereichs abhängt.

15. Röntgenvorrichtung (1) zum Abbilden eines Bereichs von Interesse eines Abbildungsbereichs der Vorrichtung (1), die Folgendes aufweist:
eine Röntgenquelle (5) zum Emittieren eines Röntgenstrahls;
wenigstens einen Kollimator (10, 11) zum Formen des Röntgenstrahls;
einen Röntgen-Zeilendetektor (12) mit einer Vielzahl von Detektionselementen, die in der Breiten- und Längsrichtung angeordnet und zum Empfangen des geformten Röntgenstrahls konfiguriert sind;
eine Speichereinheit, die konfiguriert ist, um wenigstens eine Trajektorie für den Kollimator und für wenigstens die eine Röntgenquelle und/oder die Röntgenabbildungsvorrichtung für eine Scanabbildung zu speichern;
einen Manipulator, der konfiguriert ist, um eine Öffnung des wenigstens einen Kollimators (10, 11) und wenigstens die eine Röntgenquellen (5) und/oder den Röntgen-Zeilendetektor (12) entlang der wenigstens einen Trajektorie zu verschieben; und
eine Bildrekonstruktionseinheit;
wobei die Vorrichtung (1) konfiguriert ist zum:
Durchführen einer ersten Scanabbildung eines Bereichs von Interesse eines Abbildungsbereichs mit der Öffnung des wenigstens einen Kollimators (10, 11) und wenigstens einen Röntgenquelle (5) und/oder des Röntgen-Zeilendetektors (12) einer ersten Scantrajektorie der wenigstens einen gespeicherten Trajektorie folgend;
Sammeln einer Vielzahl von Einzelbildern aus einem Belichtungsprofil der ersten Scanabbildung des Bereichs von Interesse des Patienten, wobei das Belichtungsprofil die Überlappung der Vielzahl von Einzelbildern ist, die während der ersten Scanabbildung als Funktion der Position der Vielzahl von Einzelbildern während der ersten Scanabbildung aufgenommen wurden;
Bestimmen einer Position von wenigstens einer Kante eines bestrahlten Bereichs auf wenigstens einem Einzelbild des Bereichs von Interesse des Abbildungsbereichs aus der Vielzahl von Einzelbildern aus der ersten Scanabbildung;
Herstellen einer Kantenpositionskurve in Bezug auf die erste Scanabbildung des Bereichs von Interesse des Patienten, wobei die Kantenpositionskurve eine Position einer Kante des bestrahlten Bereichs auf einem Einzelbild als Funktion der Position des Einzelbilds darstellt;
Zuschneiden ausgewählter Einzelbilder der Vielzahl von Einzelbildern aus der ersten Scanabbildung auf der Grundlage der Kantenpositionskurve; **gekennzeichnet durch**
Berechnen eines tatsächlichen Belichtungsprofils basierend auf der Kantenpositionskurve und dem Belichtungsprofil; und
Rekonstruieren des Bereichs, der für den Abbildungsbereich von Interesse ist, durch Kombinieren der zugeschnittenen ausgewählten Einzelbilder unter Verwendung des tatsächlichen Belichtungsprofils.

## Revendications

1. Procédé d'imagerie céphalométrique par rayons X d'une région d'intérêt d'un patient avec un appareil à rayons X (1), comprenant :
une source de rayons X (5) pour émettre un faisceau de rayons X ;
au moins un collimateur (10, 11) pour mettre en forme le faisceau de rayons X ;
un détecteur de ligne de rayons X (12) comportant une pluralité d'éléments de détection disposés en réseau dans la direction de la largeur et dans la direction de la longueur pour recevoir le faisceau de rayons X mis en forme ;
une unité de stockage configurée pour stocker au moins une trajectoire pour le collimateur et pour au moins un élément parmi ladite source de rayons X (5) et ledit détecteur de ligne de rayons X (12) pour une imagerie à balayage ;
un manipulateur pour déplacer une ouverture dudit au moins un collimateur (10, 11) et au moins un élément parmi ladite source de rayons X (5) et ledit détecteur de ligne de rayons X (12) le long de ladite au moins une trajectoire ; et
une unité de reconstruction d'image ;
le procédé comprenant les étapes suivantes :
la mise en œuvre (202 ; 308 ; 408) d'une première imagerie par balayage d'une région d'intérêt d'un patient avec l'ouverture dudit au moins un collimateur (10, 11) et ledit au moins un élément parmi ladite source de rayons X (5) et ledit détecteur de ligne de rayons X (12) suivant une première trajectoire de balayage de ladite au moins une trajectoire stockée ;
la collecte (308 ; 408) d'une pluralité de trames à partir d'un profil d'exposition de ladite première imagerie par balayage de la région d'intérêt dudit patient, où le profil d'exposition est le recouvrement de la pluralité de trames prises pendant une première imagerie par balayage en fonction de la position de la pluralité de trames pendant la première imagerie par balayage ;
la détermination (204 ; 310 ; 410) d'un emplacement d'au moins un bord d'une zone irradiée sur au moins une trame de la région d'intérêt dudit patient parmi ladite pluralité de trames obtenue par ladite première imagerie par balayage ;
l'établissement (206 ; 312 ; 412) d'une courbe de position de bord relative à la première imagerie par balayage de la région d'intérêt dudit patient, où la courbe de position de bord représente une position d'un bord de la zone irradiée sur une trame en fonction de la position de la trame ;
le recadrage (208 ; 314 ; 414) des trames sélectionnées de ladite pluralité de trames obtenue par ladite première imagerie par balayage sur la base de ladite courbe de position de bord ;
**caractérisé en ce que** le procédé comprend en outre les étapes suivantes :
le calcul (210 ; 316 ; 416) d'un profil d'exposition réel sur la base de la courbe de position de bord et du profil d'exposition ; et
la reconstruction (212 ; 318 ; 418) de la région d'intérêt dudit patient en combinant lesdites trames sélectionnées recadrées en utilisant ledit profil d'exposition réel.

2. Procédé selon la revendication 1, comprenant en outre le calibrage de la première imagerie par balayage de l'appareil à rayons X en :
mettant en œuvre (302 ; 402) une imagerie par balayage vide sans le patient avec l'ouverture dudit au moins un collimateur (10, 11) ayant un profil de largeur prédéterminé et au moins un élément parmi ladite source de rayons X (5) et ledit détecteur de ligne de rayons X (12) suivant ladite première trajectoire de balayage ;
collectant (302 ; 402) une pluralité de trames à partir de l'imagerie de balayage à vide ;
déterminant (304 ; 404) l'emplacement d'au moins un bord de la zone irradiée sur au moins une trame de la pluralité de trames obtenue par l'imagerie de balayage à vide ; et
l'établissement (306 ; 406) d'une courbe de position de bord d'étalonnage relative à l'imagerie par balayage à vide ;
et dans lequel la courbe de position de bord relative à la première imagerie par balayage de la région d'intérêt dudit patient est établie sur la base de la courbe de position de bord d'étalonnage et dudit emplacement d'au moins un bord d'une zone irradiée sur ladite au moins une trame de la région d'intérêt dudit patient.

3. Procédé selon la revendication 2, dans lequel l'étalonnage de la première imagerie par balayage de l'appareil à rayons X (1) est effectué au moment de l'installation dudit appareil à rayons X (1) sur le site dentaire ou avant la première imagerie par balayage de la région d'intérêt dudit patient.

4. Procédé selon la revendication 1, dans lequel ledit appareil à rayons X (1) est un appareil d'imagerie céphalométrique.

5. Procédé selon la revendication 4, dans lequel ledit au moins un collimateur (10, 11) est situé du côté du détecteur de ligne de rayons X.

6. Procédé selon la revendication 5, dans lequel ledit au moins un collimateur (10, 11) est un collimateur à lame, un collimateur à obturateur, ou une plaque avec une ouverture à fente prévue dans celle-ci.

7. Procédé selon la revendication 5, dans lequel un collimateur céphalométrique (10) et le détecteur de ligne de rayons X (12) glissent le long de deux rails parallèles.

8. Procédé selon la revendication 7, dans lequel les mouvements du collimateur céphalométrique (10) et du détecteur de ligne de rayons X (12) sont synchronisés dans un rapport de vitesse constant ordonné.

9. Procédé selon la revendication 4, dans lequel un deuxième collimateur (11) est placé devant la source de rayons X (5) et dans lequel le mouvement de l'ouverture du deuxième collimateur (11) est synchronisé avec le mouvement d'un collimateur céphalométrique (10) et du détecteur de ligne de rayons X (12).

10. Procédé selon la revendication 1, dans lequel ledit au moins un bord s'étend dans une direction transversale par rapport à la direction de balayage.

11. Procédé selon la revendication 10, dans lequel ledit au moins un bord s'étend dans une direction orthogonale à la direction de balayage, et dans lequel la source tourne pendant la première imagerie par balayage.

12. Procédé selon la revendication 1, dans lequel la largeur de la zone recadrée varie parmi les trames.

13. Procédé selon la revendication 2, dans lequel l'appareil à rayons X est un appareil panoramique (7) avec la source (5) et le détecteur de ligne de rayons X (12) placés l'un en face de l'autre sur les deux extrémités d'un portique rotatif (3), ledit au moins un collimateur (10, 11) étant un collimateur variable placé devant la source de rayons X (5) et dans lequel un profil de largeur de collimateur est stocké dans l'unité de stockage.

14. Procédé selon la revendication 13, dans lequel la largeur de la zone recadrée varie parmi les trames, et dans lequel la largeur de la zone recadrée dépend de la largeur de la zone irradiée.

15. Appareil à rayons X (1) pour imager une région d'intérêt d'une zone d'imagerie de l'appareil (1), comprenant :
une source de rayons X (5) pour émettre un faisceau de rayons X ;
au moins un collimateur (10, 11) pour mettre en forme le faisceau de rayons X ;
un détecteur de ligne de rayons X (12) comportant une pluralité d'éléments de détection disposés en réseau dans la direction de la largeur et dans la direction de la longueur et configuré pour recevoir le faisceau de rayons X mis en forme ;
une unité de stockage configurée pour stocker au moins une trajectoire pour le collimateur et pour au moins un élément parmi ladite source de rayons X et ledit dispositif d'imagerie à rayons X pour une imagerie à balayage ;
un manipulateur configuré pour déplacer une ouverture dudit au moins un collimateur (10, 11) et au moins un élément parmi ladite source de rayons X (5) et ledit détecteur de ligne de rayons X (12) le long de ladite au moins une trajectoire ; et
une unité de reconstruction d'image ;
l'appareil (1) étant configuré pour :
mettre en œuvre une première imagerie par balayage d'une région d'intérêt d'une zone à imager avec l'ouverture dudit au moins un collimateur (10, 11) et ledit au moins un élément parmi ladite source de rayons X (5) et ledit détecteur de ligne de rayons X (12) suivant une première trajectoire de balayage de ladite au moins une trajectoire stockée ;
collecter une pluralité de trames à partir d'un profil d'exposition de ladite première imagerie par balayage de la région d'intérêt dudit patient, dans lequel le profil d'exposition est le recouvrement de la pluralité de trames prises pendant une première imagerie par balayage en fonction de la position de la pluralité de trames pendant la première imagerie par balayage ;
déterminer un emplacement d'au moins un bord d'une zone irradiée sur au moins une trame de la région d'intérêt de ladite zone d'imagerie parmi ladite pluralité de trames obtenue par ladite première imagerie par balayage ;
établir une courbe de position de bord relative à la première imagerie par balayage de la région d'intérêt dudit patient, où la courbe de position de bord représente une position d'un bord de la zone irradiée sur une trame en fonction de la position de la trame ;
recadrer des trames sélectionnées de ladite pluralité de trames obtenue par ladite première imagerie par balayage sur la base de ladite courbe de position de bord ;
**caractérisé en ce que** l'appareil est en outre configuré pour :
calculer un profil d'exposition réel sur la base de la courbe de position de bord et du profil d'exposition ; et
reconstruire la région d'intérêt de ladite zone d'imagerie en combinant lesdites trames sélectionnées recadrées en utilisant ledit profil d'exposition réel.
